# EUROPEAN PATENT SPECIFICATION

(11) **EP 3 655 112 B1**
(45) Date of publication and mention of the grant of the patent: **03.09.2025**
(21) Application number: 18745859.1
(22) Date of filing: 17.07.2018
(51) Int. Cl.: A61Q 19/00, A61K 8/99, A61Q 7/00

(54) **USE OF A BACTERIAL EXTRACT FOR A COSMETIC OR DERMATOLOGICAL TREATMENT, IN PARTICULAR A MOISTURIZING TREATMENT**
VERWENDUNG EINES BAKTERIENEXTRAKTES FÜR EINE KOSMETISCHE ODER DERMATOLOGISCHE BEHANDLUNG, INSBESONDERE EINE FEUCHTIGKEITSBEHANDLUNG
UTILISATION D'UN EXTRAIT BACTÉRIEN POUR UN TRAITEMENT COSMÉTIQUE OU DERMATOLOGIQUE, EN PARTICULIER POUR UN TRAITEMENT HYDRATANT

(30) Priority: 20.07.2017 FR 1756882
(43) Date of publication of application: 27.05.2020
(73) Proprietor: SEDERMA, 78610 Le-Perray-en-Yvelines (FR)
(72) Inventor: GRACIOSO, Olga, 78610 Le Perray-en-Yvelines (FR); RINGENBACH, Caroline, 78610 Le Perray-en-Yvelines (FR); DORIDOT, Emmanuel, 78610 Le Perray-en-Yvelines (FR); DE BAENE, Frédéric, 78610 Le Perray-en-Yvelines (FR)
(74) Representative: Munier, Mélanie
(86) International application number: PCT/EP2018/069403
(87) International publication number: WO 2019/016212

(56) References cited:
- WO-A1-2004/103332
- DEL MORAL A ET AL: "Compatible solutes in new moderately halophilic isolates", FEMS MICROBIOLOGY LETTERS, WILEY-BLACKWELL PUBLISHING LTD, GB, vol. 122, no. 1-2, 15 September 1994 (1994-09-15), pages 165 - 172, XP023971511, ISSN: 0378-1097, [retrieved on 19940915], DOI: 10.1111/J.1574-6968.1994.TB07160.X
- G DENNIS SPROTT ET AL: "Identification of sulfoquinovosyl diacylglycerol as a major polar lipid in Marinococcus halophilus and Salinicoccus hispanicus and substitution with phosphatidylglycerol", CANADIAN JOURNAL OF MICROBIOLOGY, vol. 52, no. 3, 1 March 2006 (2006-03-01), CA, pages 209 - 219, XP055456265, ISSN: 0008-4166, DOI: 10.1139/w05-112

## Description

### TECHNICAL FIELD

The present invention relates to a bacterial extract and its use for a cosmetic or dermatological treatment, especially a moisturizing treatment, and more particularly a treatment of keratosis pilaris.

It concerns the cosmetics, personal care and hygiene products and dermatology industries.

### BACKGROUND ART

These industries are always in demand for ingredients to heal or beautify the skin and its appendages such as hair and nails, and the scalp. Moisturizing actives are among the most requested and represent very large sales volumes. They are indeed used at all ages, by any public, and at the same time for a preventive and curative purposes.

It is known that the skin tends to dry out due to environmental, psychological or hormonal factors. However, it is important that the skin be well hydrated and does not suffer loss of water that might cause and dryness. Skin dehydration is manifested by tightness, loss of flexibility and elasticity, and sometimes redness. The skin becomes rough, even squamous. Treating cutaneous dryness is an essential issue: allowing the *stratum corneum* to retain its flexibility and its barrier function, is also a more global way of protecting the underlying dermis and preventing skin aging.

A dry skin profile, if coupled with a skin abnormality of clogged pores, can lead to a benign condition, the keratosis pilaris.

Pilaris keratosis, quite common in men and women, results from hyperkeratosis. On certain areas of the body such as the legs, upper arms, waist and sometimes cheeks or buttocks, the skin is covered with small unsightly blisters, clearly visible, palpable, often without itching and not necessarily erythematous, which make it appear like a "goose bumps".

This mild skin disorder affects 40% of the world's female population of all ages, although more commonly seen at puberty and during pregnancy. It is characterized by an accumulation of corneocytes too abundantly produced in and at the exit of the hair follicle forming a keratinized plug which blocks and then dilates the hair follicle.

It is favored and amplified by a dry environment. Thus, keratosis pilaris is also more common in people with dry skin (ichthyosis vulgaris and atopic dermatitis), with a less effective skin barrier, less retention of epidermal water.

It is therefore a problem of keratinization of dry skin. The problem is solved by improving the process of maturation of the epidermis that restores its homeostasis. The keratinocytes of the upper part of the epidermis should be conducted to transform themselves more effectively into corneocytes to form an effective skin barrier. This makes it possible to regulate the production of moisturizing elements and lipids of the *stratum corneum,* to improve osmolyte flow, to maintain good skin hydration and thus to reduce the effects of keratosis pilaris.

### An effective skin barrier

The epidermal floor, called the basal layer, continuously produces new keratin-rich cells, called keratinocytes, that migrate to the outside of our body and undergo a major transformation throughout this migration. Keratinocytes arrive at the granular layer and produce structuring or moisturizing protein granules and precursor lipids for ceramides used to seal the skin or control its hydration. The keratinocytes produce and bind cornification proteins, such as involucrine and loricrin, with transglutaminase. They also manufacture moisturizing compounds from filaggrin, then they lose their nucleus and release the lipids between the cells. Loricrin and filaggrin are strongly diminished in keratinocytes deficient in the CALML5 protein thus indicating the crucial role of this protein. Then the corneocytes, strongly attached to each other by protein bonds, are detached from each other and produce the scales.

Thus, normal skin provides a protective function with respect to transepidermal water loss (TEWL). Surface cutaneous lipids in humans are a bulwark between the body and the environment. Any quantitative or qualitative abnormality of these lipids leads to an increase in the insensitive water loss (TEWL), a marker of the alteration of the barrier function.

Therefore, the barrier function is not complete without the indispensable contribution of various lipids, perfectly architected between them, which are produced by the keratinocytes of the granular layer, whose granules carry the protein ABCA12 (or ABC transporter A12). The latter fills its granules with ceramide precursors, allows their transport and their deposition between the keratinocytes of the *stratum corneum*, thanks to the capture and hydrolysis of ATP. Ceramides represent 50% of the lipids of the barrier. Mutations of ABCA12, by reducing ceramide intakes by up to 50%, lead to disorders of keratinization and alterations of the barrier function by the disorganization of the lipid architecture of the *stratum corneum.*

### Skin hydration and osmolytes

Water gradient drops rapidly in the epidermis from 70% in the spinous layer to 30% at the granular layer-*stratum corneum* interface and 15% at the surface. At the granular level, fluctuations in ambient humidity greatly modulate the proteolysis of filaggrin in its moisturizing by-products, which can lead to its collapse, because the enzymes in this process require an optimum of intracellular hydration.

To continue to ensure their role in the survival of the individual and reduce water loss, keratinocytes are equipped with sensors and osmolarity control systems consisting of membrane channels regulating the molecular exchanges between the inside and the outside of the cell. TAUT, one of these channels, allows the spinous and especially granular cells, the most exposed to dehydration, to concentrate taurine, a water retaining osmolyte. TAUT expression is stimulated by keratinocyte maturation and osmotic shock. Myoinositol is also an osmolyte of interest for the epidermis; its flows are managed by SMIT1 (sodium-myoinositol cotransporter-1). Like TAUT, SMIT is induced in the human keratinocyte by hyperosmosis which causes the entry of their respective osmolytes.

Aquaporin-3 (AQP3) is another of these carrier channels; it is the aquaporin most expressed by the viable epidermis, including granular layer. This membrane protein is part of a family that allows the movement of water in the cell, but AQP3 also controls the transmembrane flows of glycerol, an osmolyte known for its moisturizing properties in the epidermis. Mice deficient in AQP-3 have less water in their *stratum corneum.* It was also observed a delay in syntheses of lipids of the *stratum corneum,* an increase of the TWEL and a delay of reconstitution of the barrier after abrasion.

Furthermore, one of the important compounds for maintaining hydration of the epidermis is a very large negatively charged carbohydrate polymer: hyaluronic acid (or hyaluronan or hyaluronate). It is produced by keratinocytes, in smaller quantities compared to dermis, and is positioned around each cell where it plays a role in the solvation of vitamins and electrolytes. Hyaluronic acid ensures the capture of a very large number of water molecules: up to 1000 times its own weight. This allows the transfer of vitamins and solutes because the epidermis is not vascularized.

WO2004/103332 disclosed an extract of *Salinicoccus hispanicus* culture from inactivated cells or bacterial cell membranes.

### SUMMARY OF THE INVENTION

The aim of the present invention is to propose an active agent, more particularly a moisturizer, which will act in the most complete possible way on the various biological mechanisms described above involved in the treatment of dry skin, such as the sensible water loss, the reinforcement of the cutaneous barrier, and the osmolytes responsible for hydration.

Another aim of the invention is to provide an active having a more specific action on keratosis pilaris, the cutaneous dryness constituting a favorable ground for the development of this unsightly skin disorder.

To this aim, the present invention provides a cosmetic or dermatological active ingredient comprising a bacterial extract according to claim 1. Preferably this extract is an aqueous extract.

By intracellular extract is meant the bacterial fraction soluble in the external liquid medium, obtained after the lysis of the bacteria and the separation by centrifugation of soluble and insoluble fractions (membranes, debris ...).

The bacterium *Salinicoccus hispanicus* lives in moderately saline environments. It belongs to the phylum of firmicutes that have a strong ability to adapt to changes in their environment. *Salinicoccus hispanicus* for example survives in a highly desiccant and saline environment.

Surprisingly, the Applicant has discovered that *Salinicoccus hispanicus* in culture is able to withstand salt stress by producing an original set of molecules that can be used in cosmetics and dermatology to treat, improve or embellish the condition of the skin, its appendages (body hair, hair, nails, etc.), and scalp.

Another advantage of this bacterium is its low impact on the environment, since it only uses water and natural substrates for its growth and its cultivation is done at room temperature, which limits the consumption of energy.

The intracellular extract of *Salinicoccus hispanicus* according to the invention is characterized by the composition of its dry matter mainly containing proteins (amino acids to proteins), significant amounts of mineral salts and nucleotides, and traces of sugars and lipids.

The bacterial extract according to the invention contains:
- between 40% and 70% of proteins; preferably between 50% and 60%;
- between 20% and 40% of mineral salts; preferably between 25% and 35%;
- between 5% and 20% of nucleotides; preferably between 10% and 15%;
- 0.1% to 0.5% of lipids; preferably 0.3 to 0.4%; and
- between 0.05 and 0.15% of sugars.

The subject matter of the invention consists of a cosmetic or dermatological active ingredient containing the intracellular extract of *Salinicoccus hispanicus* according to the invention defined above and a physiologically acceptable medium. Preferably, the physiologically acceptable medium is hydrophilic in nature.

Another subject matter of the invention is a cosmetic or dermatological composition according to claim 4, and optionally one or more additional active ingredients, in a physiologically acceptable medium. The additional active agents are preferably chosen from: compounds of vitamin B3, niacinamide, tocopherol, retinol, hexamidine, α-lipoic acid, resveratrol, DHEA, hyaluronic acid, a moisturizing and/or emollient product, and one or more peptides, which are active ingredients usually used in cosmetics.

The moisturizing and/or emollient product may be chosen from plant extracts and vegetable butters/vegetable oils, fatty acid esters, squalanes and ceramides, extracts of animal origin, sugar derivatives, probiotics and prebiotics, alpha-glucans, oligosaccharides and glycols.

**In** the composition according to the invention, the active ingredient may be in a free form and optionally in a bound form, incorporated or adsorbed on macro-, micro-, and nanoparticles, or on macro-, micro- and nanocapsules, for the treatment of textiles, natural or synthetic fibers, wool, and any material intended to come into contact with the skin, such as, for example, day or night underwear, wipes, tissues, or fabrics.

Afurther subject matter of the invention relates to the use according to claim 8.

*In vitro* and *in vivo* tests described below show that the bacterial extract according to the invention is particularly effective for treatment of skin (including the scalp) which is dry or exhibits keratosis pilaris, in particular through smoothing and/or hydration of the skin and/or scalp. The skin is visibly more beautiful. Its grain is refined, more homogeneous thanks to a smoothing effect of the micro-relief. Feelings of tightness, discomfort, and redness that may or may not be associated with dry skin, are also eliminated and skin aging that is too rapid is avoided through hydration and strengthening of the dermal-epidermal junction (DEJ). The extract according to the invention may for example be used for a moisturizing treatment post-depilatory or post-peeling. According to the invention, this treatment involves in particular reinforcing the function of the cutaneous barrier and/or the regulation of the ionic fluxes in the cells of the skin and/or the protection of these cells with respect to the osmotic shocks and/or stimulation of the synthesis of laminin 5 and collagen 7, two of the major proteins of DEJ. DEJ separates the dermis from the epidermis and plays an important role in the organization and structuring of the epidermis and therefore in the surface state of the skin. In addition to its role as a support for anchoring the epidermis to the dermis, DEJ determines the adhesion of basal keratinocytes to DEJ proteins in a well-defined orientation. It also ensures transmission of proliferation and differentiation signals from the dermis to the epidermis.

A disorganization of the DEJ therefore has adverse consequences at the epidermal level and impacts the barrier function. It is also considered as one of the causes of skin aging.

Preferably, the active ingredient is used at a concentration of 0.01% to 20% by weight relative to the total weight of the composition, more preferably at a concentration of 0.1 to 5%.

The active ingredient according to the invention or said composition according to the invention is disclosed for the treatment of a disease of the skin and/or scalp. Preferably, said treatment is carried out topically.

The *in vitro* and *in vivo* tests detailed below in the description demonstrate the beneficial effects of the cosmetic treatment according to the invention, in particular on keratosis pilaris:
- Improvement of epidermal homeostasis by an action on the formation and maturation of the *stratum corneum* through the stimulation of several markers of keratinocyte differentiation: involucrine, loricrin, transglutaminase, CALML5 and ABCA12 (*in vitro* tests);
- Improvement of hydration of the epidermis by increasing the production of osmolytic transporters TAUT, SMIT1, AQP3 and hyaluronic acid (*in vitro* tests);
- Reinforcement of DEJ by stimulating the production of two of its main constituents: laminin 5 and collagen 7 (*in vitro* tests);
- Improvement of skin hydration on dry skin with or without keratosis pilaris (*in vivo* tests);
- Improvement of the microrelief (smoothing effect) on dry skin with or without keratosis pilaris (*in vivo* tests);
- Improvement of the barrier function by measuring the TEWL on dry skin with or without keratosis pilaris (*in vivo* tests);
- Reduction of keratosis pilaris on skin having such (*in vivo* tests).

"Physiologically acceptable medium" means according to the present invention, without limitation, an aqueous or aqueous-alcoholic solution, a water-in-oil emulsion, an oil-in-water emulsion, a microemulsion, an aqueous gel, an anhydrous gel, a serum, a dispersion of vesicles or a powder.

"Physiologically acceptable" means that the compositions are suitable for topical use in contact with the skin and scalp of mammals and more particularly of human, without risk of toxicity, incompatibility, instability, allergic response, among others. This "physiologically acceptable medium" forms what is usually called the excipient of the composition.

In a composition according to the invention, the bacterial extract may be combined with other active ingredients at effective concentrations that can act synergistically or in a reinforcing manner to achieve the desired effects described for the invention, such as the following agents: filtering radiation, in particular UVA and/or UVB, moisturizing, humectant, calming, myorelaxing, slimming, restructuring, firming, plumping, tensing, anti-dandruff, acting on the microcirculation, acting on inflammation, on free radicals, anti-wrinkles, lightening, acting on the radiance of the complexion, anti-glycation, propigmenting, acting on the *stratum corneum,* on the dermis-epidermis junction, on the production of protein HSPs, on the firmness, the elasticity, the tonicity of skin, regrowth of hair, peptides, vitamins etc.

The treatment according to the invention can be applied to all parts of the body, and more specifically according to the indication recommended on the face, body, neckline or scalp, in any form or vehicle known to the skilled persons in the art, particularly in the form of a solution, dispersion, emulsion, paste or powder, individually or in premix or be conveyed individually or premixed with vectors such as macrocapsules, microcapsules or nanocapsules, macrospheres, microspheres, or nanospheres, liposomes, oleosomes or chylomicrons, macroparticles, microparticles or nanoparticles, macro-sponges, micro-sponges or nanosponges, microemulsions or nanoemulsions, or adsorbed on polymers powdery organic, talcs, bentonites, spores or exines and other mineral or organic carriers.

In cosmetics in particular, applications can be proposed especially in the skincare ranges of the face, body and scalp.

CTFA ("International Cosmetic Ingredient Dictionary & Handbook" (17th Edition, 2017) published by "The Cosmetic, Toiletry, and Fragrance Association, Inc.", Washington, DC) describes a wide variety, without limitation, of cosmetic ingredients usually used in the skincare and scalp care industry, which are suitable for use as additional ingredients in the compositions of the present invention.

Other additional skin care actives that are particularly useful can be found in Sederma's commercial literature and at www.sederma.com.

The following commercial actives can also be mentioned as examples: betain, glycerol, Actimoist Bio 2^{™} (Active organics), AquaCacteen^{™} (Mibelle AG Cosmetics), Aquaphyline^{™} (Silab), AquaregulK^{™} (Solabia), Carciline^{™} (Greentech), Codiavelane^{™} (Biotech Marine), Dermaflux^{™} (Arch Chemicals, Inc), Hydra'Flow^{™} (Sochibo), Hydromoist L^{™} (Symrise), RenovHyal^{™} (Soliance), Seamoss^{™} (Biotech Marine), Argireline^{™} (nom commercial de l'acétyl hexapeptide-3 from Lipotec), spilanthol or an extract of *Acmella oleracea* known under the trade name Gatuline Expression^{™}, an extract of *Boswellia serrata* known under the name Boswellin^{™}, Deepaline PVB^{™} (Seppic), Syn-AKE^{™} (Pentaphann), Ameliox^{™}, Bioxilift^{™} (Silab), PhytoCellTec^{™}Argan (Mibelle), Papilactyl D^{™} (Silab), Preventhelia^{™} (Lipotec), or one or more of the following active ingredient marketed by Sederma : Subliskin^{™}, Venuceane^{™}, Moist 24^{™}, Vegesome Moist 24^{™}, Essenskin^{™}, Juvinity^{™}, Revidrat^{™}, Resistem^{™}, Chronodyn^{™}, Kombuchka^{™}, Chromocare^{™}, Calmosensine^{™}, Glycokin factor S^{™}, Biobustyl^{™}, Idealift^{™}, Ceramide 2^{™}, Ceramide A2^{™}, Ceramide HO3^{™}, Legance^{™}, Intenslim^{™}, Prodizia^{™}, Beautifeye^{™}, PacifeelTM, Zingerslim^{™}, Meiritage^{™}, Senestem^{™}, Sebuless^{™}, Majestem^{™}, Apiscalp^{™}, Rubistem^{™}, CitystemTM, Neonyca^{™}, NG Insaponifiables de Beurre de Karité^{™}, Majestem^{™}, or mixture thereof.

Among plant extracts (in the form of conventional extracts or prepared by an *in vitro* method) that can be combined with the bacterial extract according to the invention, there may more particularly be mentioned extracts of Ivy, in particular English Ivy (*Hedera helix*), of *Bupleurum chinensis*, of *Bupleurum falcatum,* of arnica (*Arnica montana L*), of rosemary (*Rosmarinus officinalis N*), of marigold (*Calendula officinalis*), of sage (*Salvia officinalis L*), of ginseng (*Panax ginseng*), of ginko biloba, of St.-John's-Wort (*Hyperycum perforatum*), of butcher's-broom (*Ruscus aculeatus L*), of European meadowsweet (*Filipendula ulmaria L*), of big- flowered Jarva tea (*Orthosiphon stamincus benth*), of artichoke (*Cynara scolymus*), of algae (*Fucus vesiculosus*), of birch (*Betula alba*), of green tea, of cola nuts (*Cola nipida*), of horse-chestnut, of bamboo, of *Centella asiatica,* of heather, of fucus, of willow, of mouse-ear, of escine, of cangzhu, of *chrysanthellum indicum*, of the plants of the *Armeniacea* genus, *Atractylodis platicodon*, *Sinnomenum*, *Pharbitidis*, *Flemingia*, of *Coleus* such as *C*. *Forskohlii*, *C. blumei*, *C. esquirolii*, *C. scutellaroides*, *C. xanthantus* and *C*. *Barbatus*, such as the extract of root of *Coleus barbatus,* extracts of *Ballote*, of *Guioa*, of *Davallia*, of *Terminalia*, of *Barringtonia*, of *Trema*, of *antirobia*, *cecropia*, *argania*, *dioscoreae* such as *Dioscorea opposita* or Mexican, extracts of *Ammi visnaga*, of *Siegesbeckia*, in particular *Siegesbeckia orientalis*, vegetable extracts of the family of *Ericaceae*, in particular bilberry extracts (*Vaccinium angustifollium*) or *Arctostaphylos uva ursi*, *aloe vera*, plant containing sterols (e.g., phytosterol), Manjistha (extracted from plants of the genus *Rubia*, particularly *Rubia cordifolia*), and Guggal (extracted from plants of the genus *Commiphora*, particularly *Commiphora mukul*), kola extract, chamomile, red clover extract, Piper methysticum extract (Kava Kava^{™} from Sederma), *Bacopa monieri* extract (Bacocalmine^{™} from Sederma) and sea whip extract, extracts of *Glycyrrhiza glabra,* of mulberry, of *melaleuca* (tea tree), of *Larrea divaricata*, of *Rabdosia rubescens*, of *Euglena gracilis*, of *Fibraurea recisa Hirudinea*, of *Chaparral Sorghum*, of sun flower extract, of *Enantia chlorantha*, of Mitracarpe of *Spermacocea* genus, of *Buchu barosma*, of *Lawsonia inermis L.*, of *Adiantium capillus-veneris L.*, of *Chelidonium majus,* of *Luffa cylindrica*, of Japanese Mandarin (*Citrus reticulata Blanco* var. *unshiu*), of *Camelia sinensis*, of *Imperata cylindrica*, of *Glaucium Flavum,* of *Cupressus sempervirens,* of *Polygonatum multiflorum,* of *loveyly hemsleya*, of *Sambucus nigra,* of *Phaseolus lunatus,* of *Centaurium,* of *Macrocystis pyrifera*, of *Turnera diffusa*, of *Anemarrhena asphodeloides*, of *Portulaca pilosa*, of *Humulus lupulus,* of *Coffea arabica,* of *Ilex paraguariensis*, or of *Globularia cordifolia,* of *Albizzia julibrissin*, of *Oxydendron arboretum,* of *Zingimber zerumbet smith*, of *Astragalus membranaceus*, of *Atractylodes macrocephalae,* of *Plantago lanceolata,* of *Leontopodium alpinum,* of *Mirabilis jalapa,* of *Marrubium vulgare,* or of orchids.

The compositions of the present invention may include one or more peptides, including, without limitation, di-, tri-, tetra-, penta-and hexapeptides and their derivatives. According to a particular embodiment, the concentration of the additional peptide, in the composition, ranges from 1x10⁻⁷% and 20%, preferably from 1x10⁻⁶% and 10%, preferably between 1x10⁻⁵% and 5% by weight.

According to the present invention, the term "peptide" refers to peptides containing 10 amino acids or less, their derivatives, isomers and complexes with other species such as a metal ion (e.g. copper, zinc, manganese, magnesium, and others). The term "peptides" refers to both natural peptides and synthetic peptides. It also refers to compositions that contain peptides and which are found in nature, and/or are commercially available.

Suitable dipeptides for use herein include but are not limited to Carnosine (βAH), YR, VW, NF, DF, KT, KC, CK, KP, KK, TT, PA, PM or PP.

Suitable tripeptides for use herein include, but are not limited to RKR, HGG, GKH, GHK, GGH, GHG, KFK, KAvaK, KβAK, KAbuK, KAcaK, KPK, KMOK, KMO₂K (MO₂ being a di-oxygenated sulfoxide methionine), KVK, PPL, PPR, SPR, QPA, LPA or SPA.

Suitable tetrapeptides for use herein include but are not limited to RSRK (SEQ ID NO: 1), GQPR (SEQ ID NO: 2), KTFK (SEQ ID NO: 3), KTAK (SEQ ID NO: 4), KAYK (SEQ ID NO: 5) or KFYK (SEQ ID NO: 6).

Suitable pentapeptides include but are not limited to KTTKS (SEQ ID NO: 7). Suitable hexapeptides include but are not limited to GKTTKS (SEQ ID NO: 8) and VGVAPG (SEQ ID NO: 9).

Other suitable peptides for use herein include but are not limited to: lipophilic derivatives of peptides, preferably palmitoyl (Pal) derivatives, and metal complexes as aforementioned (e.g. copper complex of the tripeptide HGG). Preferred dipeptides include for example N-Palmitoyl-β-Ala-His, N-Acetyl-Tyr-Arg-hexadecylester (Calmosensine^{™}, Idealift^{™} from Sederma), Pal-RT or Pal-KT (Sederma). Preferred tripeptide derivatives include for example Pal-GKH and Pal-GHK (from Sederma), the copper derivative of HGG (Lamin^{™} from Sigma), Lipospondin (N-Elaidoyl-KFK) and its analogs of conservative substitution, N-Acetyl-RKR-NH₂ (Peptide CK+), N-Biot-GHK (from Sederma), Pal-KAvaK, Pal-KβAlaK, Pal-KAbuK, Pal-KAcaK, or Pal-KMO₂K (Matrixyl^{®}synthe'6^{®} from Sederma), PalKVK (Syn-Coll^{™} of DSM), and derivatives thereof.

Here can further be cited also the propigmenting and/or pro-mec dipeptides and tripeptides of general formula X-(Xaa₁)n-Pro*-Xaa₂-Y disclosed in WO2014/080376, with n=0, 1 or 2, Xaa₁ an hydrophobic aminoacid selected from Ala, Val, Met, Leu, Iso, Phe, Pro, and analogs and derivatives thereof; or a polar aminoacid selected from Ser, Thr, Tyr, Asp, Glu and analogs and derivatives thereof; and when n=2 the two aminoacids Xaa₁ being the same or different; Xaa₂ being an hydrophobic aminoacid selected from Ala, Val, Met, Leu, Iso, Phe, and analogs and derivatives thereof, or a basic aminoacid selected from Arg, Lys, His, and analogs and derivatives thereof; at the N terminal end X being selected from H, -CO-R₁ and -SO₂-R₁; at the C terminal end Y being selected from OH, OR₁, NH₂, NHR₁ or NR₁R₂; R₁ and R₂ being, independently from each other, selected from an alkyl, aryl, aralkyl, alkylaryl, alkoxy et aryloxy group, that can be linear, branched, cyclic polycyclic, saturated, unsaturated, hydroxylated, carbonylated, phosphorylated and/or sulfured, said group having or not an O, S and/or N heteroatom in its skeleton and Pro* corresponding to a Proline, analog or derivative thereof; comprising for example the following peptides Pal-SPR-OH, Pal-PPR-OH, Pal-QPA-OH, Pal-LPAOH, Myr-SPA-OH, Pal-PM-OH, Pal-PA-OH and Pal-PP-OH.

Suitable tetrapeptide derivatives for use according to the present invention include, but are not limited to, Pal-GQPR (SEQ ID NO: 10) (from Sederma) and Pal-KTFK (SEQ ID NO: 11) or Ela-KTFK (SEQ ID NO: 12), Ela-KTAK (SEQ ID NO: 13), Ela-KAYK (SEQ ID NO: 14) or Ela-KFYK (SEQ ID NO: 15). Suitable pentapeptide derivatives for use herein include, but are not limited to, Pal-KTTKS (SEQ ID NO: 16) (available as Matrixyl^{®} from Sederma), Pal-YGGFXaa (SEQ ID NO: 17) with Xaa being Leu or Pro, or mixtures thereof. Suitable hexapeptide derivatives for use herein include, but are not limited to, Pal-VGVAPG (SEQ ID NO: 18), Pal-GKTTKS (SEQ ID NO: 19), Pal-HLDIIXaa with Xaa being Trp, Phe, Tyr, Tic, 7-hydroxy-Tic ou Tpi (SEQ ID NO: 20) and derivatives thereof. The mixture of Pal-GHK and Pal-GQPR (SEQ ID NO: 10) (Matrixyl^{®} 3000, Sederma) can also be mentioned. The preferred compositions commercially available containing a tripeptide or a derivative include Biopeptide-CL^{™}, Maxilip^{™}, Biobustyl^{™}, Procapil^{™} and Matrixyl^{®}synthe'6^{®} of Sederma. The compositions commercially available preferred sources of tetrapeptides include Rigin^{™}, Eyeliss^{™}, Matrixyl^{®} Reloaded and Matrixyl 3000^{®} which contain between 50 and 500 ppm of Pal-GQPR (SEQ ID NO: 10) and an excipient, proposed by Sederma.

The following marketed peptides can be mentioned as well as additional active ingredients:
- Vialox^{™} (INCI name = Pentapeptide-3 (synthetic peptide comprising alanine, arginine, isoleucine, glycine and proline)), Syn-ake^{™} (β-Ala-Pro-Dab-NH-Bzl) or Syn-Coll^{™} (Pal-Lys-Val-Lys-OH) marketed by Pentapharm;
- Argireline^{™} (Ac-Glu-Glu-Met-Gln-Arg-Arg-NH₂ (INCI name = Acetyl hexapeptide-3) (SEQ ID NO: 21), Leuphasyl^{™} (Tyr-D-Ala-Gly-Phe-Leu) (SEQ ID NO: 22), Aldenine^{™} (Gly-His-Lys), Trylagen^{™} (INCI name = Pseudoalteromonas Ferment Extract, Hydro lyzed Wheat Protein, Hydro lyzed Soy Protein, Tripeptide-10 Citrulline (reaction product of Citrulline and Tripeptide-10 (synthetic peptide constituted of aspartic acid, isoleucine and lysine)), Tripeptide-1), Eyeseryl^{™} (Ac-β-Ala-His-Ser-His)(SEQ ID NO: 23), Serilesine^{™} (Ser-Ile-Lys-Val-Ala-Val) (SEQ ID NO 24) or Decorinyl^{™} (INCI name: Tripeptide-10 Citrulline = reaction product of Citrulline and Tripeptide-10 (synthetic peptide constituted of aspartic acid, isoleucine and lysine) marketed by Lipotec;
- Collaxyl^{™} (Gly-Pro-Gln-Gly-Pro-Gln (SEQ ID NO 25)) or Quintescine^{™} (Cys-Gly) marketed by Vincience;
- Cytokinol^{™}LS (casein hydrolysate) marketed by Les Laboratoires Serobiologiques/Cognis;
- Kollaren^{™} (Gly-His-Lys), IP2000^{™} (Pal-Val-Tyr-Val) or Meliprene^{™} (INCI name = Monofluoroheptapeptide-1: reaction product of acetic acide and a synthetic peptide comprising arginine, glycine, glutamic acid, histidine, norleucine, p-fluorophenylalanine and tryptophan) marketed by l'Institut Européen de Biologie Cellulaire;
- Neutrazen^{™} (Pal-His-D-Phe-Arg-NH₂) marketed by Innovations; or
- BONT-L-Peptide^{™} (INCI name = Palmitoyl Hexapeptide-19: reaction product of palmitic acid and Hexapeptide-19 (synthetic peptide constituted of asparagine, aspartic acid, lysine and methionine), Timp-Peptide^{™} (INCI name = Acetyl Hexapeptide-20: reaction product obtained by acetylation of Hexapeptide-20 (synthetic peptide constituted of alanine, glycine, lysine, valine and proline) or ECM Moduline^{™} (INCI name = Palmitoyl Tripeptide-28: reaction product of palmitic acid and Tripeptide-28 (synthetic peptide constituted of arginine, lysine and phenylalanine) marketed by lnfinitec Activos.

More specifically, according to the invention the alkyl-phthalide(s) or plant extract comprising such may be combined with at least one of compounds selected from compounds of the vitamin B3, compounds such as niacinamide or tocopherol, retinoid compounds such as retinol, hexamidine, α-lipoic acid, resveratrol or DHEA, hyaluronic acid, peptides, in particular N-acetyl-Tyr-Arg-O-hexadecyl ester, Pal-VGVAPG (SEQ ID NO: 18), Pal-KTTKS (SEQ ID NO: 16), Pal-GHK, Pal-KMO₂K and Pal-GQPR (SEQ ID NO: 10), which are widely used active ingredients in topical cosmetic or dermopharmaceutical compositions.

More specifically, the bacterial extract according to the invention can be combined with a moisturizing and/or emollient product for the skin, its appendages (nails, eyelashes, eyebrows), the scalp, the hair, in particular one or more compounds below:
- Plant extracts & vegetable butters/vegetable oils, for example:
   - a butter of shea (and unsaponifiable), mango, cocoa and/or avocado, oil of coconut and or *Moringa oleifera*,
   - an oil of *Rosa moschata*, *evening primrose*, camellia, sunflower, oat bran, *Vitis vinifera* seeds, babassu, bamboo, aloe vera, apricot, olive, sweet almond and/or argan.
   - an extract of *Imperata cylindrica* roots, rice, *Calendula officinalis* flower, cinnamon, green tea, *Porphyridium cruentum*, *Helianthus annuus* seeds, *Achillea millefolium*, and/or seaweed (*Spirulina platensis*)
- Fatty acid esters, for example:
   - the commercial products markedted by CRODA: Crodamol ISIS^{™} (INCI name "Isostearyl Isostearate"), Duraquench IQ^{™} (INCI name "Cetyl Alcohol (and) Isostearyl Isostearate (and) Potassium Cetyl Phosphate (and) Cetyl Stearate (and) Stearic Acid "), Cromolient DP3A^{™} (INCI name" Di-PPG-3 Myristyl Ether Adipate ").
   - Saqualans and ceramids.
- Extracts of animal origin, for example:
   - Omega 3 and Omega 6, essential fatty acids, snail slime, beeswax, honey, lanolin, collagen and allantoin, for example.
- Sugar derivatives, for example:
   - trehalose, sorbitol, xylitol and/or lactitol
   - PCA sodium (sodium salt of pyrrolidone carboxylic acid), methyl gluceth-20 (methyl beta-D-glucoside ethoxylated), DMDM Hydantoin, Acetamidopropyl Trimonium Chloride (Incrometant), Ubiquinone, Panthenol, Lactate (derivative).
- Probiotics (Lactobacillus) and prebiotics, alpha-glucans, and/or oligosaccharides
- Glycols (for example: glycerol, butylene glycol, propylene glycol, polyethylene glycol, and derivatives, etc.).

The present invention also provides a cosmetic topical treatment method for improving the appearance and general condition of the skin and scalp, including topical application to the skin of a subject in need thereof of an effective amount of at least one bacterial extract according to the invention or a composition comprising it, in a physiologically acceptable excipient.

By "topical treatment" or "topical use" is meant an application that is intended to act at the place where it is applied: skin or scalp.

A composition according to the invention can be applied locally to the targeted areas, for example using a canula-type applicator suitable for the scalp.

The "effective" amount depends on a variety of factors, such as age, condition of the patient, severity of the disorder or condition and the way of administration. An effective amount means a non-toxic amount sufficient to achieve the desired effect.

All percentages and ratios used in this application are by weight of the total composition and all measurements are made at 25°C unless otherwise specified.

For example, for a cosmetic facial treatment, the European Cosmetics Directive has set a standard application amount of a cream of 2.72 mg/cm²/day/person and for a lotion for the body of 0.5 mg/cm²/day/person.

According to other features, the cosmetic treatment method according to the invention may be associated with one or more other treatment methods for the skin, such as, for example, light therapy, heat or aromatherapy treatments.

According to the invention, it is possible to propose devices with several compartments or kits intended for the implementation of the method described above, and which could include, by way of example, and without being limiting, in a first compartment a composition containing at least the bacterial extract and in a second compartment an additional excipient and/or active, the compositions contained in said first and second compartments being here considered as a combination composition for simultaneous, separate or spread over time use, especially in one of the treatments defined above.

The treatment method according to the invention is more particularly suitable for a hydration treatment, smoothing, soothing, a treatment for reducing redness and feelings of tightness and cutaneous discomfort, especially post-depilation or combined with an exfoliating or post exfoliation treatment, and/or a treatment of keratosis pilaris, and/or a treatment intended to slow down skin aging.

### DETAILED DESCRIPTION

The present invention will be better understood in light of the following detailed description and illustration examples.

### A) Example of preparing a bacterial extract of Salinicoccus hispanicus within the frame of the present invention

The *Salinicoccus hispanicus* strain is cultured in a fermentation medium comprising in particular sugars, proteins (peptone, yeast extract) and highly enriched in salts (especially NaCl). The salt content can range from 10 to 100 g/l.

The temperature of the culture may be between 20 and 40°C.

Additions of compounds are possible at different times in the culture.

The culture is stopped when the OD (optical density) at 600 nm reaches between 30 and 40 (measurement of turbidity).

The assembly bacteria + medium is then centrifuged, the bacteria washed with water and the fresh biomass recovered in 10% of the initial total volume (concentration of the biomass by a factor of 10). The bacteria are then lysed either mechanically or chemically. The intracellular content is thus released into the external environment. Envelopes and cell debris are removed by centrifugation. The pH of the supernatant is adjusted to 7. The supernatant is filtered.

The filtrate constitutes said "extract according to the invention" used hereinafter.

This bacterial extract can be characterized by the composition of its dry matter:
- Protides (amino acids to proteins): about 60%
- Mineral salts (sodium, potassium, phosphates, sulphates and chlorides): about 30%
- Nucleotides: about 12%
- Lipids: 0.3%
- Sugars: 0.1%

### B) Formulation of an active ingredient according to the invention

The active ingredient comprises the extract according to the invention obtained according to A) above pure or diluted in a matrix forming a physiologically acceptable medium. This active ingredient is especially intended for the cosmetic industry for the preparation of cosmetic products, creams, gels, etc.

(see galenic examples in point D) below). The active ingredient will preferably be formulated between 0.1 and 10% in a cosmetic composition applicable to the skin, preferably at a few%, especially 2 to 5%, depending on the more or less concentrated effect desired.

The dilution of the extract according to the invention can be carried out in any physiologically acceptable hydrophilic excipient. Water and water-miscible solvents of the type, for example glycerin or any other physiologically acceptable short chain polyol, will preferably be used.

For example, for the formulas of the galenic of point D), which will be used for the *in vivo* tests, it is a dilution by a factor 4 which has been preferentially used to manufacture the active ingredients.

For the *in vitro* tests, the pure extract according to the invention will be used in the appropriate aqueous medium of each test.

### C) Galenic

Various cosmetic formulations are described below. Additional active ingredients, acting in support and/or in addition to the activity of the active ingredient according to the invention may be added in the appropriate phase according to their hydrophobic or hydrophilic nature. These ingredients can be of any category according to their function(s), the place of application (scalp, face, body, neck, bust, hands, etc.), the desired end effect and the targeted consumer.

The active ingredient according to the invention used in the galenical formulations given below is the extract according to the invention diluted by a factor of 4 in an aqueous excipient.

For the *in vitro* tests on skin explants and *in vivo* tests, the cream 1) described below was used.
**1) Cream form**

| **Ingredient/INCI name** | **Role** | **% w/w** |
|---|---|---|
| **Part A** | | |
| H₂O (Aqua). | - | qsp100 |
| Carbomer (carbopol Ultrez 10) | Rheology modifier | 0.20 |

| **Part B** | | |
|---|---|---|
| Arlacel^{™} 170 (Glyceryl Stearate & PEG-100 Stearate) | Emulsifier O/W | 1.00 |
| Crodacol^{™} CS90 (Cetearyl Alcohol) | Viscosity additive | 1.50 |
| Crodamol^{™} GTCC(Caprylic /Capric Triglyceride) | Emollient | 3.00 |

| **Part C** | | |
|---|---|---|
| Crodamol^{™} AB (C12-15 Alkyl Benzoate) | Emollient | 1.50 |
| Pemulen^{™} TR2 (Acrylates/C10-30 Alkyl Acrylate Crosspolymer) | Rheology modifier | 0.20 |

| **Part D** | | |
|---|---|---|
| Glycerin (Glycerin) | Humectant | 2.00 |
| Octanediol (Caprylyl Glycol) | Biocide | 0.50 |

| **Part E** | | |
|---|---|---|
| Phenoxyethanol | Preservative | qs |

| **Part F** | | |
|---|---|---|
| Potassium sorbate | Preservative | qs |

| **Part G** | | |
|---|---|---|
| H₂O (Aqua) | - | 4.00 |
| Sodium hydroxyde 30% | pH ajuster | 0.40 |

| **Part H** | | |
|---|---|---|
| Active ingredient according to the invention | Biological active | 3.00 |

| **Part I** | | |
|---|---|---|
| Flagrance | Flagrance | 0.10 |

**2) Oil form**

| **Ingredient/INCI name** | **Role** | **% w/w** |
|---|---|---|
| **Part A** | | |
| Crodamol^{™} OP (Ethylhexyl Palmitate) | Emollient | qsp100 |
| Crodamol^{™} GTCC (Caprylic /Capric Triglyceride) | Emollient | 10.00 |
| Cropure^{™} Sweet Almond (Prunus Amygdalus Dulcis | Vegetable oil | 5.00 |
| Oil) | | |
| Crodamol^{™} AB (C12-15 Alkyl Benzoate) | Light emollient | 5.00 |
| Crodamol^{™} DA (Diisopropyl Adipate) | Light emollient | 5.00 |
| Crodamol^{™} STS (PPG-3 Benzyl Ether Myristate) | Satin emollient | 1.00 |
| Dl Alpha Tocopherol | Antioxydant | 0.20 |

| **Part B** | | |
|---|---|---|
| Phenoxyethanol | Preservative | 0.80 |

| **Part C** | | |
|---|---|---|
| Cithrol^{™} 10GTIS (PEG-20 Glyceryl Triisostearate) | Emulsifier | 15.00 |
| Active ingredient according to the invention | Biological active | 4.00 |

**3) Serum form**

| **Ingredient/INCI name** | **Role** | **% w/w** |
|---|---|---|
| **Part A** | | |
| H₂O (Aqua) | - | qsp100 |
| Potassium sorbate | Preservative | 0.10 |

| **Part B** | | |
|---|---|---|
| Glycerin | Humectant | 8.00 |
| Phenoxyethanol | Preservative | 0.80 |
| Keltrol^{™} CG-SFT (Xanthan Gum) | Rheology modifier | 0.20 |
| Cyamopsis Tetragonoloba (Guar) Gum) | Rheology modifier | 0.10 |
| Satiagel^{™} VPC 512 (Chondrus Crispus (Carrageenan) Extract) | Rheology modifier | 0.70 |

| **Part C** | | |
|---|---|---|
| Tween^{™} 20 (Polysorbate 20) | Solubilizer | 1.00 |

| **Part D** | | |
|---|---|---|
| Active ingredient according to the invention | Biological active | 3.00 |

| **Part E** | | |
|---|---|---|
| H₂O (Aqua) | - | 0.10 |
| Lactic acid | pH ajuster | 0.02 |

**4) Exfoliant cream form**

| **Ingredient/INCI name** | **Role** | **% w/w** |
|---|---|---|
| **Part A** | | |
| H₂O (Aqua) | - | qsp 100 |
| Citric acid | pH ajuster | 0.30 |
| Sodium citrate | pH ajuster | 0.80 |
| Potassium sorbate | Preservative | 0.10 |
| Sodium sulfite | Antioxydant | 0.01 |

| **Part B** | | |
|---|---|---|
| Glycerin | Humectant | 4.00 |
| Phenoxyethanol | Preservative | 0.80 |
| Keltrol^{™} CG-SFT (Xanthan Gum)⁵ | Rheology modifier | 0.50 |
| Cyamopsis Tetragonoloba (Guar) Gum) | Rheology modifier | 0.30 |
| Satiagel^{™} VPC 512 (Chondrus Crispus (Carrageenan) Extract) | Rheology modifier | 0.90 |
| Cromollient^{™} SCE (Di-PPG-2 Myreth-10 Adipate) | Emollient | 1.00 |

| **Part C** | | |
|---|---|---|
| Crodasinic^{™} LS30 (Sodium Lauroyl Sarcosinate & Cleansing agent Aqua) | 20.00 | |
| Crodesta^{™} SL40 (Aqua & Sucrose Cocoate & Alcohol) Cleansing agent | 5.00 | |

| **Part D** | | |
|---|---|---|
| Active ingredient according to the invention Biological active | 3.00 | |

| **Part G** | | |
|---|---|---|
| Apricot Exfoliator 10007 (Prunus Armeniaca Seed Particles Powder) | 0.50 | |

### Examples of active ingredients marketed by SEDERMA that can be added to this formulation:

- EVERMAT^{™}: association of an extract of *Enantia chlorantha* rich in protoberberins and oleanolic acid, which reduces pore size and shine, and refines skin grain.
- CALMOSENSINE^{™}: soothing active for sensitive skins comprising the Tyr-Arg lipo-dipeptide. It reduces discomfort feelings.
- KELISOFT^{™}: active ingredient based on vegetable chelidonine which in particular slows hair growth and calms post-hair removal inflammation.
- Ceramide 2^{™}: active ingredient consisting of a pure substance identical to the ceramides present in the skin, which constitute 40 to 50% of the skin lipids. This ingredient has a restructuring function and maintains hydration and integrity of the skin barrier.
- NG Birch Sap^{™}: raw sap from birch sapwood; skin toning and moisturizing.
- Shea Butter: having nourishing and protective properties for the treatment of skin stressed by the environment.
- NG UNSAPONIFIABLE SHEA BUTTER^{™}: active ingredient with nourishing and protective properties for the treatment of skin damaged by the environment.
- PACIFEEL^{™}: active ingredient comprising a natural extract of the *Mirabilis jalapa* plant also known as the Marvel of Peru, which alleviates cutaneous discomfort, fades redness of sensitive and reactive skin and strengthens and hydrates the epidermis.
- REVIDRATE^{™}: active (Myristyl Phosphomalate) that in particular improves the cohesion of the epidermis and its hydration.
- RIGIN^{™}: active improving the elasticity and firmness of the skin, increasing hydration and smoothing the skin.
- SEBULESS^{™}: purifying sebo-regulator ingredient comprising a *Syringa vulgaris* extract, which mattifies and refreshes complexion, fades the inflammatory blemishes.
- SUBLISKIN^{™}: active ingredient that moisturizes and smooths the skin while allowing it to resist to external aggressions (based on acetylated glucuronic acid oligosaccharides).
- DERMAXYL^{™}: anti-aging active ingredient which smoothes wrinkles and repair the skin barrier.

### D) In vitro test results

A first series of tests focused on the reinforcement and maturation of the barrier through the stimulation of several markers of keratinocyte differentiation.

A second series of tests focused on improving the hydration of the epidermis through the stimulation of hyaluronic acid and osmolytes transporters.

A third series of tests focused on strengthening the JDE, through the stimulation of two of its main structural proteins: collagen 7 and laminin 5.

A final test has shown the interest of the product to stimulate the production of ATP and thus to bring energy to the skin cells.

The *in vitro* tests below were performed on several different biological models all of human origin: keratinocytes and skin explants from cosmetic surgery. The extract according to the invention, as prepared in point A) above, was tested either directly at the indicated concentrations (for cell culture tests) or integrated into the cream 1) described above in the Galenic part C) (for explant tests).

### 1. Reinforcement and maturation of the barrier

### Effect of the extract according to the invention on the differentiation of keratinocytes

### Principle

Normal human keratinocytes almost at confluence are brought into contact with the extract according to the invention (between 0.125 and 0.75%) in a suitable culture medium in order to study their differentiation under a microscope by following the appearance of the particular layers using varied and appropriate microscopic techniques.

### Results

No hyperproliferation of KH was observed with the extract according to the invention. With the extract according to the invention, a clear acceleration of the differentiation with respect to the control is observed. This results in the rapid onset (4 days) of typical structures of the upper layers of the epidermis (presence of branched structures characteristic of the rigid proteolipid matrix and in
multilayer of the horny envelope; characteristic intracellular network). At the same time of observation, the control mat does not have such a differentiated profile.

Thus, the bacterial extract according to the invention is able to stimulate the differentiation of KH in order to establish an effective barrier.

### Differentiation markers

Dry skins, and in particular those of persons prone to keratosis pilaris, are characterized by deficient levels of intercellular lipids at the level of the *stratum corneum* (including in particular ceramides and neutral lipids). This contributes to the weakening of the cutaneous barrier. In addition, involucrin, loricrin, filaggrin, transglutaminase and CALML5 are among the proteins responsible for the good quality of the barrier function of the *stratum corneum,* allowing "normal" skin to limit water losses at level of the barrier.

Therefore, in order to improve dry skin, cosmetic active agents capable of stimulating the production and/or the expression of these proteins and/or the production of these lipids will be sought.

On the same mats as those used above to observe differentiation, a number of differentiation markers were evaluated.

### Principle

The same culture and contact protocol as previously was used on human keratinocytes. The keratinocytes were then labeled by immunocytochemistry using antibodies specific to markers of cornification/maturation of these cells: involucrin, loricrin, filaggrin and ceramide-2. Reproducible photos were made and quantified by image analysis, in comparison with the control.

Moreover, the increase in the expression of transglutaminase-1 but also the transglutaminase activity and the amount of neutral lipids produced by KH in culture in contact with the extract according to the invention were measured and compared with those of their control without product. The methods were respectively: qRT-PCR, enzyme-substrate enzymatic activity assay and red oil labeling of neutral lipids included in KH.

Finally, the cream 1) described above in Galenic part C) was applied to skin explants (female, 50 years old, abdomen) which were then sectioned to be immunohistologically labeled to evaluate CALML5. The photos taken were then analyzed by image analysis and compared to placebo cases.

### Results on involucrin and loricrin

**Table 1: Variation of the expression of involucrin and loricrin by KH in the presence of the bacterial extract according to the invention; Immunocytochemistry, n=3.**

| | **Involucrin (AFU)** | ***Variation*** | **Loricrin (AFU)** | ***Variation*** |
|---|---|---|---|---|
| Solvent control | 26.1 +/- 26.5 | ***Reference*** | 10.8 +/- 13.2 | ***Reference*** |
| **0.125% of the invention extract** | 43.7 +/- 33.9 | ***+68%; p<0.05*** | 86.0 +/- 84.0 | ***+695%; p<0.01*** |
| **0,25% of the invention extract** | 117.0 +/- 40.3 | ***+349%; p<0.01*** | 108.8 +/- 64.0 | ***+906%; p<0.01*** |
| **0,75% of the invention extract** | 154.2 +/- 87.6 | ***+491%; p<0.01*** | 109.5 +/- 91.6 | ***+912%; p<0.01*** |

| | | | | |
|---|---|---|---|---|
| *AFU: Arbitrary fluorescence units* | | | | |

The results show the stimulating effect of the bacterial extract according to the invention on the maturation of KH in culture. There is a higher expression of the protein involucrine in KH (+491%; *p*<*0.01*), as well as loricrin protein (+912%; *p<0.01*) which are respectively weakly expressed or almost absent in their case control.

### Results on filaggrin and ceramide-2

Table 2: Variation of the expression of filaggrin and ceramide-2 by KH in the presence of the bacterial extract according to the invention; Immunocytochemistry, n=3.

| | **Filaggrin (AFU)** | ***Variation*** | **Ceramide-2 (AFU)** | ***Variation*** |
|---|---|---|---|---|
| Solvent control | 16.1 +/- 31.5 | ***Reference*** | 1.02 +/- 0.94 | ***Reference*** |
| **0.125% of the extract according to the invention** | 53.5 +/- 38.4 | ***+232%; p<0,01*** | 8.47 +/- 8.24 | ***+727%; p<0.01*** |
| **0.25% of the extract according to the invention** | 59.8 +/- 29.8 | **+*271%; p<0,01*** | 12.42 +/- 17.72 | ***+1114%; p<0.01*** |

The results confirm the stimulatory effect of the bacterial extract on the maturation of KH in culture. Thus, a higher expression of the filaggrin protein in KH (+271%, *p<0.01)* and ceramide-2 lipid (+1114%, p <0.01) is also observed, both being almost absent in control cases.

### Results on neutral lipids and transglutaminase-1

**Table 3: Variation of mRNA expression and transglutaminase activity in KH in the presence of the bacterial extract according to the invention; n = 4.**

| | **Transglutaminase-1 (qRT-PCR)** | ***Variation*** | **Transglutaminase (AFU/10³cell.)** | ***Variation*** |
|---|---|---|---|---|
| Control | 1.00 +/- 0.03 | ***Reference*** | 157.5 +/- 26.9 | ***Reference*** |
| **0.25% of the extract according to the invention** | / | / | 259.4 +/-41.8 | ***+65%; p<0.01*** |
| **0.5% of the extract according to the invention** | 1.58 +/- 0.05 | ***+58%; p<0.01*** | 270.2 +/- 27.3 | ***+72%; p<0.01*** |

The results show a higher production of neutral lipids in KH (x 8.9; *p<0.01),* even though they are almost absent in the control case.

These results show that the bacterial extract according to the invention effectively promotes the expression of transglutaminase-1 mRNAs (+ 58%; *p<0.01).* The complementary study on the intracellular activity of transglutaminases shows an increase (+ 72%; *p <0.01*) which confirms the effect of the bacterial extract according to the invention on these cells.

### Results on CALML5

The cream 1) described above in Galenic part C), applied for 6 consecutive days on 3 skins, increased + 79.4% the signal corresponding to CALML5 compared to the placebo cream applied on an equivalent number of control skins. This increase is significant at-risk *p<0.01.*

In conclusion, it appears that the extract according to the invention stimulates all the tested markers of the maturation of the *stratum corneum,* with a view to reinforcing the skin barrier effect.

### 2. Improvement of skin hydration

### Principle

The beneficial effects of the bacterial extract according to the invention on the regulation of hydration have been studied with several models.

Firstly, skin explants (woman, 38 years, abdomen): the two creams, the one containing the extract according to the invention (cream 1) described above in Galenic part C), the other its placebo, were applied to the *stratum corneum* daily for 6 days. At the end of this step, the skins were cut into thin sections (8µm) and the DSG-1 protein was labeled by immunohistology. On cuts from the same trial, AQP-3, a major water and glycerol transporter, was also immunohistologically labeled and quantified by image analysis on photos. The markings obtained with the cream containing the extract according to the invention were compared with those obtained with the placebo cream.

In a second series of tests, the extract according to the invention, in solution in the culture medium, was brought into contact with human keratinocytes (KH) and then a hyaluronic acid assay was performed by a method derivative from Elisa on culture media.

In a third series of tests, the ability of the extract according to the invention to promote mRNA production of two other cell-important osmolyte transporters, TAUT and SMIT-1, was assessed by qRT-PCR. After contact with the extract according to the invention, human keratinocytes were crushed and mRNAs corresponding to these proteins were evaluated by qRT-PCR after conversion into cDNA.

In the same way, the expression of the mRNAs of the ABCA12 protein was evaluated.

### Results on DSG-1 & AQP3

**Table 4: Modulation of DSG-1 and AQP3 after contact with a 0.75% cream of the extract-based active ingredient according to the invention or its placebo; skin explants; immunohistology, n = 5 or 6.**

| | **DSG-1 (AFU)** | ***Variation*** | **AQP3 (AFU)** | ***Variation*** |
|---|---|---|---|---|
| Control | 12.41 +/- 6.12 | ***Reference*** | 21.87 +/- 5.62 | ***Reference*** |
| **0.75% of the extract according to the invention** | 17.68 +/- 5.70 | **+42.5 *%; p<0.01*** | 35.47 +/- 7.98 | ***+62.2%; p<0.01*** |

These results show that the extract according to the invention positively modulates two proteins important for the water homeostasis of the skin: DSG1 and AQP3. The first to maintain cell cohesion between them and the second to manage the flow of electrolytes. There was an increase of +42.5% *(p<0.01)* for DSG-1 and +62% (*p <0.01*) for AQP3.

### Results on hyaluronic acid

**Table 5: Variation of hyaluronic acid production by KH in the presence of the extract according to the invention; Elisa (n = 4).**

| | **Hyaluronic acid (ng/10⁶ cell.)** | ***Variation*** |
|---|---|---|
| Control | 5683 +/- 635 | ***Reference*** |
| **0.25% of the extract according to the invention** | 7563 +/- 603 | **+33% ; *p<0.01*** |
| **0.5% of the extract according to the invention** | 8776 +/- 636 | ***+54% ; p<0.01*** |
| **075% of the extract according to the invention** | 10447 +/- 1518 | ***+84% ; p<0.01*** |

These results show that the extract according to the invention positively modulates the production of hyaluronic acid which is known to be deposited around these cells forming a kind of hydric bubble. The latter allows the transit of osmolytes, nutrients and vitamins. The effect is clear from 0.25% and reaches +61% *(p<0.01).*

### Results for TAUT & SMIT1

**Table 6: Variation of TAUT and SMIT-1 mRNA production by KH in the presence of the extract according to the invention; qRT-PCR, after 24 hours of contact (n = 4).**

| | **TAUT (qRT-PCR, ratio)** | ***Variation*** | **SMIT-1 (qRT-PCR, ratio)** | ***Variation*** |
|---|---|---|---|---|
| Control | 1.01 +/- 0.18 | ***Reference*** | 1.00 +/- 0.11 | ***Reference*** |
| **0.5% of the extract according to the invention** | 1.53 +/- 0.17 | **+51%; *p<0.01*** | 1.51 +/- 0.52 | **+50%; *nsd*** |
| **0.75% of the extract according to the invention** | 1.74 +/- 0.23 | ***+72%; p<0.01*** | 2.74 +/- 0.74 | **+173%; *p<0.01*** |

### nsd: non significant data

As previously, a very positive effect of the extract according to the invention for modulating the TAUT and SMIT1 proteins, known to regulate the flow of epidermal electrolytes, is noticed. TAUT mRNA expression increase was +72% (*p<0.01*) and that of SMIT1 +173% (*p<0.01*).

### Results for ABCA12

**Table 7: Variation in the production of ABCA12 mRNA by KH in the presence of the extract according to the invention; qRT-PCR (n = 4).**

| | **ABCA12 at 2h (qRT-PCR, ratio)** | ***Variation*** | **ABCA12 at 8h (qRT-PCR, ratio)** | ***Variation*** |
|---|---|---|---|---|
| Control | 1.02 +/- 0.26 | ***Reference*** | 1.01 +/- 0.20 | ***Reference*** |
| **0.75% of the extract according to the invention** | 2.19 +/- 0.62 | **+114% *; p<0.05*** | 1.74 +/- 0.09 | **+71% ; *p<0.01*** |

These results show that ABCA12 is increased early under the influence of the extract according to the invention: + 114% (2h, *p<0.05*) and + 71% (8h, *p<0.01).*

In conclusion, it appears that the extract according to the invention stimulates all the markers going in the sense of a reinforced hydration of the skin.

### 3. Strengthening the dermo-epidermal junction (DEJ)

Laminin 5 is important at the level of the DEJ. It ensures the proper anchoring of basal keratinocytes on the basement membrane and is responsible for the suppleness of the epidermis. In addition, it stimulates the proliferation of keratinocytes, allowing them to engage in differentiation. On older cells Laminin 5 is no longer replaced as efficiently as on young cells, hence the interest of stimulating the biosynthesis for a better renewal.

The type VII collagen is in the form of fibrils and allows the anchorage of the DEJ to the elastic fibers of the dermis.

### Principle

In a first series of tests, the extract according to the invention in solution in the culture medium was brought into contact with human keratinocytes. An assay of laminins and collagen VII was made on the media; the result is reduced to the number of cells present on the layers.

In a second series of tests laminin 5 was evaluated on skin explants. The two creams, the one containing the extract according to the invention: cream 1 described above in Galenic part C), the other its placebo, were applied to the *stratum corneum* every day for 5 days. At the end of this step, the skins were cut into thin sections (8µm) and the laminin 5 was labeled by immunohistology and then quantified by image analysis in photos. The labelings obtained with the cream containing the extract according to the invention were compared with those obtained with the placebo cream.

### Results

**Table 8: Variation of laminin production by KH in the presence of the extract according to the invention; Elisa (n = 4).**

| | **Laminins (ng/10⁶ cell.)** | ***Variation*** |
|---|---|---|
| Control | 269.8 +/- 9.8 | ***Reference*** |
| **0.25% of the extract according to the invention** | 397.6 +/- 15.5 | ***+47%; p<0.01*** |
| **0.75% of the extract according to the invention** | 433.5 +/- 28.4 | **+61%; *p<0.01*** |

The extract according to the invention positively modulates the production of laminins. The effect is clear from 0.25% and reaches +47% (*p<0.01*)*.*

**Table 9: Variation of the production of collagen VII by KH in the presence of the extract according to the invention; Elisa (n = 4).**

| | **Collagen VII (ng/10⁶ cell.)** | ***Variation*** |
|---|---|---|
| Control | 27 +/- 2 | ***Reference*** |
| **0,25% of the extract according to the invention** | 44 +/- 10 | ***+*64%*; p<0.01*** |
| **0,75% of the extract according to the invention** | 52 +/- 7 | **+97%*; p<0.01*** |

The extract according to the invention positively modulates the production of collagen VII. The effect is clear from 0.25% and reaches +97% *(p<0.01).*

**Table 10: Modulation of laminin 5 after contact with a cream according to the invention or its placebo; skin explants; immunohistology, n = 3 or 4.**

| | **Laminin 5 (AFU)** | ***Variation*** |
|---|---|---|
| Control | 10.35 +/- 3.29 | ***Reference*** |
| **0,75% of the extract according to the invention** | 18.01 +/- 3.30 | **+74% ; *p<0.01*** |

These results show that the extract according to the invention positively modulates laminin 5 synthesis. An increase of +74% is noted (*p<0.01*)*.*

### Conclusion

The extract according to the invention stimulates the synthesis of laminin and collagen VII favoring better anchoring of basal keratinocytes to JDE, their proliferation/differentiation and a better anchoring of the DEJ on the elastic components of the dermis.

### 4. Stimulation of ATP production :

In preliminary studies on human keratinocytes, it has been shown that 0.5 and 0.75% of the extract according to the invention strongly stimulate the neo-synthesis of ATP in these cells by respectively +55% and +148% (in both cases with *p<0.01*)*.*

Thanks to this action, the extract according to the invention allows the cells of the skin to have energy enabling them to meet at least part of their needs.

### E) In vivo tests

### Principle

An evaluation of the skin efficacy on the smoothing, hydration, restructuring, soothing, reduction of discomfort and post-stress redness of the active ingredient according to the invention used at 3% was conducted out of a total of 127 volunteers in 2 independent studies:
- A placebo-controlled study to measure the effect of the extract on dry skins, sensitive to hair removal and, where possible, provided with keratosis pilaris. Several complementary techniques have been used to study different parameters:
   ∘ An evaluation of hydration, performed by analysis of scales on standardized photographs.
   ∘ An evaluation of the barrier function following insensitive water loss.
   ∘ An evaluation of the microrelief, performed by fingerprint analysis by fringe projection
   ∘ An evaluation of keratosis pilaris by fingerprint analysis by fringe projection.
- A study to evaluate the perceived effect of 3% of the active ingredient according to the invention on the discomfort and redness after depilation, by a large panel of 104 volunteers.

### 1. Effect on dry skins having keratosis pilaris

### Protocol

This first study was carried out on a total panel of 23 women of 34 years old age (19 - 49 years), having the skin of legs dry with visible scales and feeling systematically discomfort following a depilation of the legs with wax. In addition, many of them (N = 16) had keratosis pilaris on the upper arms or thighs. Prerequisite conditions and/or during the study were imposed on the volunteers (absence of shaving, use of creams on the legs, exposure to the sun, etc.) in order to limit artifactual effects.

### Type of study, duration and applications

This study was conducted in single blind on the legs and upper arms. The volunteers applied a cream containing 3% of the ingredient according to the invention (cream according to the invention corresponding of formula 1) of point D) above) and a placebo cream (without active ingredient) in contra-lateral. The creams were applied in bi-daily massage for 4 weeks.

The synopsis of the study can be summarized according to the diagram below.

T0 Depilation T0 T1 week T4 weeks Depilation T4 weeks.

---------------------------------------------------------------------------------------------------------------------------------- >

Photos Photos Photos

Imprints Imprints

TEWL TEWL TEWL TEWL

Statistical studies were performed using Student's t-test or if needed with a non-parametric Wilcoxon test. Bilateral tests were performed on matched series. For questionnaire evaluations, a Khi2 test was used.

### Methods and results

### Evaluation of the hydration and homeostatic effect by squam analysis

Standardized photos were captured directly on a dry area of the lower leg using a dermoscopic camera using constant LED illumination and a high precision optical system to perfectly standardize colors, brightness and geometry.

At T0, T1week and T4 weeks, 2 acquisitions were made on 2 neighboring but independent sites. From the images obtained, a selective extraction of the scales was carried out using a software and a percentage of squams was obtained for each case.

**Table 11: Variation of the amount of scales after application of the cream according to the invention or of the placebo cream for 4 weeks (n = 2 sites x 20 volunteers)**

| **Scales (in% of occupation of the total surface area)** | **Cream of the invention** | | | **Placebo cream** | | |
|---|---|---|---|---|---|---|
| | T0 | T1s | T4s | T0 | T1s | T4s |
| **Mean** | 7.56 | 2.76 | 1.50 | 6.13 | 3.32 | 1.69 |
| +/- **Standard deviation** | +/- 5.72 | +/- 3.13 | +/- 2.27 | +/- 3.32 | +/- 4.06 | +/- 2.02 |
| **Variation *vs*. T0 (%)** | reference | -63.5% | -80.2% | reference | -45.8% | -72.4% |
| Significance *vs.* T0 | | *p<0.01* | *p<0.01* | | *p<0.01* | *p<0.01* |
| Maximum | | -98% | -99% | | | |
| Responders | | 90% | 100% | | | |
| Significance vs. Placebo | | *p<0.05* | *p<0.05* | | | |

The application of the cream according to the invention leads to a significant improvement, significant from 1 week, with a decrease in the amount of scales of 63.5% (*p<0.01 vs.* T0 and *p<0.05 vs.* placebo), while the application of a placebo leads to a lower decrease of 45.8%.

After 4 weeks of application of the products, a cumulative effect causes almost disappearance of the scales with -80.2% for the cream according to the invention and 72.4% for the placebo (*p<0.01* for both *vs.* T0). The difference in effect remains significant in favor of the cream according to the invention (*p<0.05*)*.*

### Evaluation of the hydration effect by quotation on photos

The standardized photos, previously captured, were appreciated by a panel of 5 expert judges. For each volunteer, the experts visualized the pictures before and after treatment (at T0, T1sem and T4sem.) And quantified the attenuation of scales according to 5 grades. For the presentation of the results, a binary grouping was carried out to separate the "significant" effects from the "negligible" effects.

**Table 12: grades and groupings used**

| **Grades** | **Groupings used** |
|---|---|
| **4: strongly** | Significant effects |
| **3: a lot** | |
| **2: moderately** | |
| **1: slighly** | Negligible effects |
| **0: no effect** | |

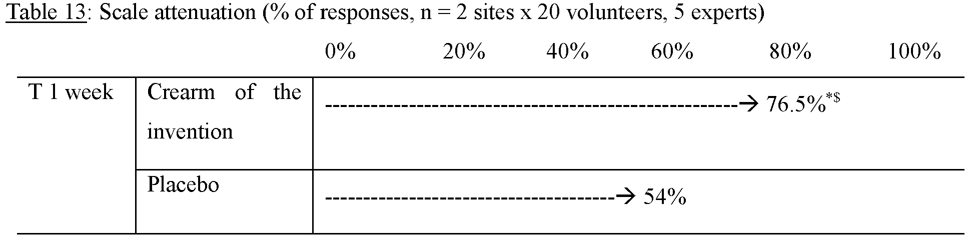

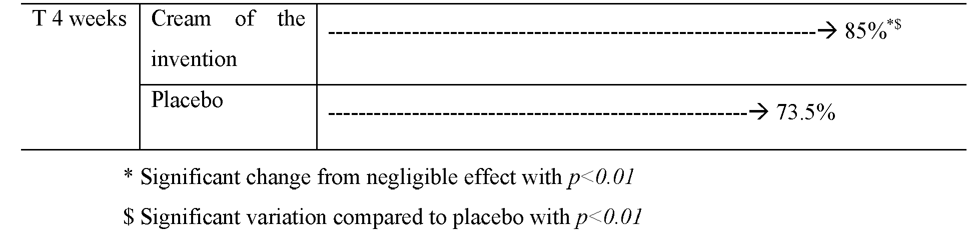

The results show that the experts see a noticeable decrease in scales from the first week of application of the cream according to the invention with an occurrence of 76.5%. This decrease is judged to be significantly greater (*p<0.01*) than that seen after placebo (54%).

After 4 weeks of use, it is noted by the experts an amplification of the skin hydrated appearance (85% significant effect), this value still remains significantly higher (*p<0.01*) to placebo (73.5%).

These results confirm those obtained by the quantification by image analysis previously carried out.

### Barrier function by the TEWL

As mentioned above, depilation is a triggering or aggravating factor of certain skin disorders including keratosis pilaris. The selected volunteers reported feeling systematically discomfort after wax depiltion of their legs. The effect of the active agent according to the invention was evaluated via a measurement of the TEWL, known to characterize the integrity of the cutaneous barrier. The measurement of the TEWL was carried out in the first (T0) and the second (T4weeks) appointments each time in two stages: before and a little after the standardized withdrawal of a few layers of the *stratum corneum* using adhesive discs (Dsquames^{®}), this stress mimic reproducibly what hair removal does.

After rupture of the homeostasis of the *stratum corneum* by this means, it is expected at four weeks of application of the cream according to the invention a lower increase of the TEWL than that observed at T0 and a smaller increase of the TEWL compared to the placebo cream. This indicates a strengthening of the *stratum corneum* and a lower sensitivity to stress.

**Table 14: Variation of the differences of TEWL observed after stress mimicking hair removal at T0 and T4weeks; effect of the cream according to the invention at 3% or of its placebo. (n = 3measures, 21 volunteers)**

| **Increased of TEWL differences after destruction in g/m2/h** | **Cream of the invention** | | **Placebo** | |
|---|---|---|---|---|
| | T0 | T4 weeks | T0 | T4 weeks |
| **Mean** | 10.5 | 4.1 | 8.6 | 5.8 |
| +/- **Standard deviation** | +/- 10.9 | +/- 3.3 | +/- 7.9 | +/- 3.1 |
| **% variation *vs.* T0** | | **-61%** | | **-32.6%** |
| Significance *vs.* T0 | | *p<0.05* | | *nsd* |
| Maximum | | -112% | | |
| Responders | | 67% | | |
| Significance vs. Placebo | | *p<0.05* | | |

The increase in TEWL observed after removal of the skin barrier by adhesives, is reduced by 61% *(p<0.05 vs.* T0 and vs. placebo, Table 11) after 4 weeks of use of the cream according to the invention. The latter promotes a better resistance of the cutaneous barrier vis-à-vis a stress miming the epilation, this being explained thanks to the observations of the *in vitro* tests and on explants. At the same time, the application of the placebo cream only resulted in insignificant and more moderate enhancement of *32% (nsd* vs. T0).

### Microrelief evaluation

The small skin disorders change the physiology of the skin which frequently results in the modification of the softness. Less smooth skin often has small dead skin called scales. At time T0 and T4week, a negative imprint of the skin of the legs was performed using Silflo^{™} silicone polymer. An acquisition was then made by the fringe projection technique (Dermatop, Eotech) on these imprints and an analysis was conducted with a specialized software.

Two representative parameters of the smoothing of the skin were analyzed:
- the overall roughness of the surface: Sq
- the % of surface occupied by the visible network (depths>15-20µm)

**Table 15: Variation of the microrelief on the legs after application of the cream according to the invention at 3% or placebo for 4 weeks. (n = 20 volunteers)**

| | **Rugosity of surface (in µm)** | | | | **% of surface occupied by the deep network (in %)** | | | |
|---|---|---|---|---|---|---|---|---|
| | **Cream of the invention** | | **Placebo** | | **Cream of the invention** | | **Placebo** | |
| | T0 | T4 weeks | T0 | T4 weeks | T0 | T4 weeks | T0 | T4 weeks |
| **Mean** | 16.9 | 15.2 | 16.4 | 16.4 | 6.11 | 4.73 | 5.53 | 5.63 |
| **+/- Standard deviation** | +/- 3.8 | +/- 3.2 | +/- 3.4 | +/- 3.2 | +/-2.81 | +/- 2.40 | +/- 2,76 | +/- 3,42 |
| **% variation *vs*. T0** | | -10% | | 0% | | -22.6% | | 1.8% |
| Significance | | *p<0.01* | | *nsd* | | *p<0.05* | | *nsd* |
| Maximum | | -31% | | | | -77% | | |
| Responders | | 75% | | | | 70% | | |
| Significance vs. Placebo | *p<0.01* | | | | *p<0.05* | | | |

The analysis of the results (Table 12) shows that the application of the cream according to the invention for 4 weeks causes a significant reduction in the depth of the folds of the microrelief and their number. Thus, the overall roughness and the% of surface occupied by the deep network are improved by respectively 10% (*p<0.01*) and 22.6% *(p<0.05).* These effects are statistically significant compared to the application of a placebo, which does not involve any significant modification of the two previous parameters (0% and 1.8%).

### Evaluation of the reduction of keratosis pilaris

Negative imprints of areas with keratosis lesions on the 16 volunteers that had some (13 on the upper thighs and 3 on the upper arm) were performed. These imprints were then processed as before.

**Table 16: Variation of keratosis pilaris after application of the cream according to the invention at 3% or placebo for 4 weeks. (n = 16 volunteers)**

| | **Amount of keratosis pilaris lesions (for a surface of 20x20mm)** | | | | **Total perimeter of keratosis pilaris lesions (in mm for a surface of 20x20mm)** | | | |
|---|---|---|---|---|---|---|---|---|
| | **Cream of the invention** | | **Placebo** | | **Cream of the invention** | | **Placebo** | |
| | T0 | T4sem | T0 | T4sem | T0 | T4sem | T0 | T4sem |
| **Mean** | 23.7 | 21.2 | 20.9 | 21.5 | 106.2 | 98.2 | 98.9 | 103 |
| **+/- Standard deviation** | +/- 7.6 | +/- 7.3 | +/- 7.2 | +/- 9.2 | +/- 29.1 | +/- 35.7 | +/-24.9 | +/- 45.5 |
| **% variation vs.T0** | | **-10.5%** | | +2.9% | | **-7.5%** | | +4.1% |
| Significance | | *p=0.07* | | *nsd* | | *nsd* | | *nds* |
| Maximum | | -39% | | | | -47% | | |
| Responders | | 56% | | | | 69% | | |
| Significance vs. Placebo | *p<0.01* | | *p<0.05* | | | | | |

The results show the attenuation of visible lesions of keratosis pilaris through the use of the cream according to the invention. The latter causes a decrease in the number of visible lesions by 10.5% (*p =0.07 vs.* T0). It is also noticed with the cream according to the invention a decrease of 7.5% of the total perimeter of the lesions which reflects their shrinkage. The comparison with placebo, which does not cause any improvement, shows that the difference is significant for these two parameters (*p<0.01* and *p<0.05*)*.*

### Analysis of the perceived effect post depilation

This study was carried out on a panel of 104 women with a mean age of 35 years (18 - 49 years old), declaring that their skin was dry and observing a feeling of discomfort and redness during wax depilation of their legs. A period without shaving or depilation was required in order to be able to perform a wax depilation during the evaluation. This study was conducted in open on the legs.

On the day of the evaluation, the volunteers proceeded with a wax depilation of the two half-legs, and immediately after, one leg received a single application of the cream according to the invention while the other leg served as a witness. The perceived effect was evaluated in the short term (between 5 and 30 minutes after application) and then in the longer term (2 hours after application), using a self-evaluation questionnaire.

For each statement, the volunteers had to choose:

| | |
|---|---|
| • Totally agree | Merged into "favorable opinions" |
| • Agree | |
| • Indifferent | |
| • Disagree | Merged into "defavorable opinions" |
| • Totally disagree | |

Furthermore, volunteers were asked if they were satisfied with the product (score from 0 to 10, only scores of 7 to 10 result in satisfaction). Statistical studies were performed using the Khi2 test comparing the% of favorable responses to the% of unfavorable responses.

The results show a very good perception of the product, with a high satisfaction rate (77%) and a preferred texture (91%) and quickly absorbed (80%). The immediate post-depilation effect is real with a moisturized skin (93%), a decrease in redness (52%) and less discomfort (65%). This feeling is confirmed 2 hours after applications with skin still hydrated (92%), a greater decrease in redness (64%) and a slight increase in comfort (67%).

## Claims

1. Cosmetic or dermatological active ingredient comprising a bacterial extract and a physiologically acceptable medium, **characterized in that** said extract is an intracellular extract of *Salinicoccus hispanicus* comprising:
- between 40% and 70% of proteins;
- between 20% and 40% of mineral salts;
- between 5% and 20% of nucleotides;
- 0.1% to 0.5% of lipids; and
- between 0.05 and 0.15% of sugars.

2. Ingredient according to claim 1, **characterized in that** said extract is aqueous.

3. Ingredient according to claim 1 or 2, **characterized in that** the physiologically acceptable medium is hydrophilic in nature.

4. Cosmetic or dermatological composition comprising an intracellular bacterial extract of *Salinicoccus hispanicus* or the active ingredient according to anyone of claims 1-3, and optionally one or more additional active ingredients in a physiologically acceptable medium.

5. Composition according to claim 4, said additional actives being chosen from: compounds of vitamin B3, niacinamide, tocopherol, retinol, hexamidine, α-lipoic acid, resveratrol, DHEA, hyaluronic acid, a moisturizing and/or emollient product, one or more peptides.

6. Composition according to claim 5, the moisturizing and/or emollient product being chosen from plant extracts and vegetable butters/vegetable oils, fatty acid esters, squalans and ceramides, extracts of animal origin, sugar derivatives, probiotics and prebiotics, alpha-glucans, oligosaccharides and glycols.

7. The composition according to anyone of claims 4-6, wherein the active ingredient is in a bound, incorporated or adsorbed form on macro-, micro-, and nanoparticles, or on macro-, micro- and nanocapsules, for the treatment of textiles, natural or synthetic fibers, wool, and any material intended to come into contact with the skin, such as day or night underwear, wipes, tissues, or tissues.

8. Use of an intracellular bacterial extract of *Salinicoccus hispanicus* or an active ingredient according to claim 1-3 or a composition according to claims 4-7, for a topical, non-therapeutic cosmetic treatment of the skin and its appendages, and scalp.

9. Use of an extract according to claim 8 for a treatment of:
- moisturization; and/or
- smoothing; and/or
- soothing; and/or
- reduced feelings of tightness and cutaneous discomfort; and/or
- reduction of redness; and/or
- intended to slow down skin aging; and/or
- keratosis pilaris.

10. Use according to claim 8 or 9, **characterized in that** the treatment will strengthen the function of the skin barrier, regulate the ionic flux in skin cells and/or protect these cells against osmotic shocks, stimulate the synthesis of two important molecules of the JDE, laminin 5 and collagen 7.

11. Use according to claims 8-10, **characterized in that** the active ingredient is used at a concentration of 0.01% to 20% by weight relative to the total weight of the composition.

12. Use of the intracellular extract of *Salinicoccus Hispanicus* and a physiologically acceptable medium to prepare a cosmetic or dermatological composition.

## Patentansprüche

1. Kosmetischer oder dermatologischer aktiver Inhaltsstoff, umfassend einen bakteriellen Extrakt und ein physiologisch akzeptables Medium, **dadurch gekennzeichnet, dass** der Extrakt ein intrazellulärer Extrakt von *Salinicoccus hispanicus* ist, umfassend:
- zwischen 40 % und 70 % Proteine;
- zwischen 20 % und 40 % Mineralsalze;
- zwischen 5 % und 20 % Nukleotide;
- 0,1 % bis 0,5 % Lipide; und
- zwischen 0,05 und 0,15 % Zucker.

2. Inhaltsstoff nach Anspruch 1, **dadurch gekennzeichnet, dass** der Extrakt wässrig ist.

3. Inhaltsstoff nach Anspruch 1 oder 2, **dadurch gekennzeichnet, dass** das physiologisch akzeptable Medium von Natur aus hydrophil ist.

4. Kosmetische oder dermatologische Zusammensetzung, umfassend einen intrazellulären bakteriellen Extrakt von *Salinicoccus hispanicus* oder den aktiven Inhaltsstoff nach einem beliebigen der Ansprüche 1-3 und optional einen oder mehrere zusätzliche aktive Inhaltsstoffe in einem physiologisch akzeptablen Medium.

5. Zusammensetzung nach Anspruch 4, wobei die zusätzlichen Wirkstoffe ausgewählt sind aus: Verbindungen von Vitamin B3, Niacinamid, Tocopherol, Retinol, Hexamidin, α-Liponsäure, Resveratrol, DHEA, Hyaluronsäure, einem feuchtigkeitsspendenden und/oder weichmachenden Produkt, einem oder mehreren Peptiden.

6. Zusammensetzung nach Anspruch 5, wobei das feuchtigkeitsspendende und/oder weichmachende Produkt ausgewählt ist aus Pflanzenextrakten und pflanzlichen Buttern/pflanzlichen Ölen, Fettsäureestern, Squalanen und Ceramiden, Extrakten tierischen Ursprungs, Zuckerderivaten, Probiotika und Präbiotika, Alpha-Glucanen, Oligosacchariden und Glykolen.

7. Zusammensetzung nach einem beliebigen der Ansprüche 4-6, wobei der aktive Inhaltsstoff in gebundener, eingearbeiteter oder adsorbierter Form auf Makro-, Mikro- und Nanopartikeln oder auf Makro-, Mikro- und Nanokapseln vorliegt, zur Behandlung von Textilien, natürlichen oder synthetischen Fasern, Wolle und allen Materialien, die dazu bestimmt sind, mit der Haut in Kontakt zu kommen, wie Tages- oder Nachtwäsche, Wischtücher, Taschentücher oder Gewebe.

8. Verwendung eines intrazellulären bakteriellen Extrakts von *Salinicoccus hispanicus* oder eines aktiven Inhaltsstoffs nach Anspruch 1-3 oder einer Zusammensetzung nach den Ansprüchen 4-7 zur topischen, nicht-therapeutischen kosmetischen Behandlung der Haut und ihrer Anhangsgebilde sowie der Kopfhaut.

9. Verwendung eines Extrakts nach Anspruch 8 zur Behandlung von:
- Feuchtigkeitsversorgung; und/oder
- Glättung; und/oder
- Beruhigung; und/oder
- verringerte Spannungsgefühle und unangenehme Hautempfindungen; und/oder
- Verringerung von Rötungen; und/oder
- bestimmt zur Verlangsamung der Hautalterung; und/oder
- Keratosis pilaris.

10. Verwendung nach Anspruch 8 oder 9, **dadurch gekennzeichnet, dass** die Behandlung die Funktion der Hautbarriere stärkt, den Ionenfluss in Hautzellen reguliert und/oder diese Zellen vor osmotischen Schocks schützt, die Synthese von zwei wichtigen Molekülen der JDE, Laminin 5 und Kollagen 7, stimuliert.

11. Verwendung nach den Ansprüchen 8-10, **dadurch gekennzeichnet, dass** der aktive Inhaltsstoff in einer Konzentration von 0,01 % bis 20 % des Gewichts, bezogen auf das Gesamtgewicht der Zusammensetzung, verwendet wird.

12. Verwendung des intrazellulären Extrakts von *Salinicoccus Hispanicus* und eines physiologisch akzeptablen Mediums zur Herstellung einer kosmetischen oder dermatologischen Zusammensetzung.

## Revendications

1. Ingrédient actif cosmétique ou dermatologique comprenant un extrait bactérien et un milieu physiologiquement acceptable, **caractérisé en ce que** ledit extrait est un extrait intracellulaire de *Salinococcus hispanicus* comprenant :
- Entre 40% et 70% de protéines ;
- Entre 20% et 40% de sels minéraux ;
- Entre 5% et 20% de nucléotides ;
- 0,1% à 0,5% de lipides ; et
- Entre 0,05 et 0,15% de sucres.

2. Ingrédient selon la revendication 1, **caractérisé en ce que** ledit extrait est aqueux.

3. Ingrédient selon la revendication 1 ou 2, **caractérisé en ce que** le milieu physiologiquement acceptable est de nature hydrophile.

4. Composition cosmétique ou dermatologique comprenant un extrait bactérien intracellulaire de *Salinococcus hispanicus* ou un ingrédient actif selon l'une des revendications 1 à 3, et éventuellement un ou plusieurs ingrédients actifs additionnels dans un milieu physiologiquement acceptable.

5. Composition selon la revendication 4, lesdits actifs additionnels étant choisis parmi : les composés de la vitamine B3, la niacinamide, le tocophérol, le rétinol, l'hexamidine, l'acide α-lipoïque, le resvératrol, la DHEA, l'acide hyaluronique, un produit hydratant et/ou émollient, un ou plusieurs peptides.

6. Composition selon la revendication 5, le produit hydratant et/ou émollient étant choisi parmi les extraits de plantes et beurres végétaux /huiles végétales, les esters d'acides gras, les squalanes et céramides, les extraits d'origine animale, les dérivés de sucre, les probiotiques et prébiotiques, les alpha-glucans, les oligosaccharides et les glycols.

7. Composition selon l'une des revendications 4 à 6, dans laquelle l'ingrédient actif se présente sous une forme liée, incorporée ou adsorbée sur des macro-, micro-, et nanoparticules, ou sur macro-, micro- et nanocapsules, pour le traitement des textiles, des fibres naturelles ou synthétiques, des laines, et tous matériaux destinés à entrer en contact avec la peau, comme par exemple les sous-vêtements de jour ou de nuit, les lingettes, les mouchoirs, ou les tissus.

8. Utilisation d'un extrait intracellulaire de *Salinococcus hispanicus,* ou d'un ingrédient actif selon l'une des revendications 1 à 3 ou d'une composition selon l'une des revendications 4 à 7, pour un traitement cosmétique topique non thérapeutique de la peau et de ces annexes, et du cuir chevelu.

9. Utilisation de l'extrait selon la revendication 8, pour un traitement :
- d'hydratation ; et/ou
- de lissage; et/ou
- apaisant ; et/ou
- de diminution des sensations de tiraillement et d'inconfort cutané ; et/ou
- de diminution de rougeurs ; et/ou
- destiné à freiner le vieillissement cutané ; et/ou
- de la kératose pilaire.

10. Utilisation selon la revendication 8 ou 9, **caractérisée en ce que** le traitement va permettre de renforcer la fonction de la barrière cutanée, réguler les flux ioniques dans les cellules de la peau et/ou protéger ces cellules vis-à-vis des chocs osmotiques, stimuler la synthèse de deux molécules importantes de la JDE, la laminine 5 et le collagène 7.

11. Utilisation selon l'une des revendications 8 à 10, **caractérisée en ce que** l'ingrédient actif est utilisé à une concentration de 0,01% à 20% en poids par rapport au poids total de la composition.

12. Utilisation d'un extrait intracellulaire de *Salinococcus hispanicus* et un milieu physiologiquement acceptable pour préparer une composition cosmétique ou dermatologique.
